# EUROPEAN PATENT APPLICATION

(11) **EP 1 393 718 A1**
(43) Date of publication of application: **03.03.2004**
(21) Application number: 02078557.2
(22) Date of filing: 29.08.2002
(51) Int. Cl.: A61K 9/107, C08G 81/02

(54) **Colloidal drug carrier system**

(71) Applicant: OctoPlus Sciences B.V., 2333 CL Leiden (NL)
(72) Inventor: Verrijk, Rudolf, 2202 HS Noordwijk (NL); Ramos, Delphine, 2316 ST Leiden (NL); Franssen, Okke, 3525 VM Utrecht (NL)
(74) Representative: Prins, Adrianus Willem

(57) **Abstract**

The present invention relates to a drug carrier system comprising a plurality of colloidal particles having a core and a shell, said particles being comprised of copolymer molecules, which copolymer comprises at least one A block and at least one B block different from the at least one A block, wherein the at least one A block consists of a polymer unit of a first set of monomers and the at least one B block consists of a second set of monomers. In addition, the invention relates to block copolymers that are useful in this system, as well as pharmaceutical compositions based on said colloidal system.

## Description

### FIELD OF THE INVENTION

The present invention relates to a drug carrier system comprising a plurality of colloidal particles, to a block copolymer useful in the preparation of such a system and to method for making said system and said block copolymer. Further, the present invention relates to a pharmaceutical composition comprising said carrier system or said block copolymer.

More specifically, the invention relates to colloidal drug carrier systems based on novel polymeric carriers. In particular, it relates to carrier systems representing crosslinked micelles formed by novel copolymers composed of hydrophilic A- and B-blocks. In another aspect, the invention provides novel copolymers which are useful for the preparation of such drug carrier systems, but also as stabilizers of aqueous two-phase systems. In a further aspect, the invention describes pharmaceutical compositions comprising colloidal drug carrier systems and methods for their preparation.

### BACKGROUND OF THE INVENTION

Over the past decade, micellar structures based on block copolymers have emerged as novel and highly promising colloidal drug carrier systems. Typically, the block copolymers from which micelles are formed, are of the A-B type, with one of the blocks being relatively hydrophilic (such as PEG, polyethylene glycol) and the other relatively hydrophobic (such as PLA, polylactide). For use of carrying and transporting drugs, these systems are developed for aqueous systems, which makes that the hydrophilic part forms the shell of the micelle, and the hydrophobic part forms the core of the micelle.

Due to their nanoscale dimensions and their unique physicochemical properties, these micellar structures have been shown to have much potential in product applications in which most conventional carrier systems have failed. For instance, poorly soluble drugs entrapped in micelles can be transported at concentrations exceeding their water solubility. With appropriately designed surface properties, micelles are capable of circulating in the blood stream for an extended period of time without being rapidly eliminated by the macrophages of the reticuloendothelial system (RES). In contrast to other nanoscale carrier systems such as liposomes or nanocrystals, micelles are formed easily, rapidly, reproducibly and with little energy consumption.

On the other hand, simple block copolymer micelles have several disadvantages, of which a few important ones are indicated in the next sentences. First of all, they are principally unstable at concentrations lower than the critical micelle concentration (CMC) of the polymer. In addition, the release properties of micelles are not controllable within the same broad range as that of other colloids: a drug is rather rapidly released as a result of its partitioning from the micelle core into the surrounding aqueous phase. Further, micelles cannot be isolated or dried and reconstituted, which makes their handling at least inconvenient.

To overcome at least some of these difficulties, functionalised micelles have been developed. One of the most important features of these improved micelle systems was the introduction of reactive groups into the micelle-forming molecules, allowing the stabilization of the micelles through chemical fixation, and particularly through intramicellar crosslinking. Crosslinked micelles behave like water-insoluble nanoparticles in that they are stable below the CMC of the micelle forming polymers. Owing to their increased stability, crosslinked micelles may have a much longer circulation time in the bloodstream than simple micelles.

In the state of the art, two classes of crosslinked micelles have been investigated more thoroughly: shell crosslinked micelles and core crosslinked micelles. As said, the block copolymers from which the micelles are formed comprise a hydrophilic (such as PEG, polyethylene glycol) block and a rather hydrophobic (such as PLA, polylactide) block. Chemical modifications introducing reactive groups, such as methacryloyl groups, were successfully carried out for either of the two block types.

For instance, Kataoka *et al.* describe in Macromolecules 32 (1999), 1140-1146, a PLA-PEG system which is modified to carry a methacryloyl group at the distal end of the PLA block. The micelles formed from the modified polymer were crosslinked thermally in the presence of a radical initiator to give core crosslinked micelles. The resulting nanoparticles were resistant to organic solvents and to detergents such as sodium dodecyl sulfate.

Alternatively, the hydrophobic block can also be modified laterally, allowing a higher degree of crosslinking and stability. However, lateral crosslinking reduces the free volume of the hydrophobic core of the micelle, resulting in a reduced drug load capacity.

Other approaches, such as the system described by Thurmond *et al.* in Nucleic Acids Res. 27 (1999), 2966-2971, have used the hydrophilic block for the introduction of polymerisable groups, leading to shell crosslinked micelles or "knedels". These structures have the advantage of providing an additional mechanism to control drug release, *i.e.* diffusion of the drug through a polymerized shell. As an extension of this concept, hollow nanocapsules were prepared by chemically removing the core of the crosslinked micelles. See in this light Huang *et al.* in J. Am. Chem. Soc. 121 (1999), 3805-3806.

These known structures based on crosslinked micelles have certain disadvantages. For instance, these systems have a limited drug load capacity due to the small particles size of the carriers and to the poor relationship between the core and the shell volumes. Especially, the capacity for hydrophilic drug substances, such as peptides, proteins, nucleic acids and polysaccharides, is very low because of the relatively hydrophobic nature of the copolymer blocks which define the core of the micelles.

Thus, there is a need to provide improved colloidal drug carriers, which overcome one or more of the disadvantages of the prior art micelle systems. Particularly, there is a need for micelle systems which allow the efficient incorporation of hydrophilic drug substances, and especially highly hydrophilic drug substances.

Further, there is a need for improved biocompatible compounds which act as micelle forming agents, especially for block copolymers which are less hydrophobic than those presently used for preparing crosslinked micelles.

### SUMMARY OF THE INVENTION

According to the invention, a colloidal drug carrier system is provided that, at least partially, meets the need sketched. This carrier system comprises colloidal particles based on a hydrophilic block copolymer having A blocks oriented towards the core and B blocks oriented towards the shell of the particles. The block copolymers themselves are preferably configured as A-B or A-B-A type copolymers. Both A and B are hydrophilic; however, they are partially incompatible in aqueous solutions. More specifically, as a physical mixture of homopolymers in water, A and B can induce phase separation, *i.e.* they are capable of forming a two-phase system in water. The hydrophilic block copolymers of the invention have been found to form self-assembled structures such as micelles. The colloidal particles and the drug carrier system of the invention are based on or derived from the self-assembled structures of such block copolymers.

In some of the preferred embodiments, the colloidal particles formed by the copolymer are chemically (intraparticulary) crosslinked, which requires that at least one of the copolymer blocks comprises a crosslinkable group or structure. Core crosslinked micelles are prepared by crosslinking A blocks, shell crosslinked micelles by crosslinking B blocks. Optionally, the core of the crosslinked micelles is expanded through the incorporation of homopolymer A to increase the particle size and load capacity.

In some embodiments, the colloidal particles are biodegradable. This property is achieved by selecting one of the copolymer blocks to be liable to biodegradation. Even without biodegradable block backbones, biodegradability can be achieved by the presence of biodegradable spacers between the polymer backbone and the intermolecular crosslinks.

In some preferred embodiments, the colloidal drug carrier system comprises particles prepared from A-B or A-B-A block copolymers of a modified dextran such as dextran hydroxyethylmethacrylate (block A) and polyethylene glycol (block B). The core of such particles is optionally expanded with an amount of the modified dextran to yield larger particles having a higher drug load capacity.

The colloidal particles are preferably prepared from a micellar solution of the respective copolymer by a crosslinking step. Alternatively, particles with an expanded core may be prepared from an aqueous two-phase system wherein the block copolymer is assembled at the interface of the dispersed inner phase and the coherent outer phase. In this case, not only the A blocks of the copolymer, but also any homopolymer A present in the dispersed phase may be crosslinked.

An advantage of the colloidal particles of the invention is their usefulness as carriers for hydrophilic drug substances, such as peptides, proteins, polysaccharides or nucleic acids. These compounds can be effectively and efficiently incorporated, as the core of the particles is not hydrophobic as in the case of conventional crosslinked micelles prepared from amphiphilic copolymers. The colloidal particles can be prepared in the presence of the drug substance in an all-aqueous process, avoiding the handling, health and environmental difficulties associated with organic solvents. Alternatively, the particles are first prepared and then loaded with the active ingredient.

Another advantage of the invention is that it provides a drug carrier system based on colloidal particles of which the diameter can be controlled within narrow limits through the selection of the process parameters during their preparation. In the case of crosslinked micelles with an expanded core, for instance, the relative amount of homopolymer A that is present in the two-phase system from which the particles are prepared by crosslinking, is a critical parameter to control the droplet size of the dispersed phase, which, in turn, controls the particle size after crosslinking.

Further embodiments, exemplifications, and advantages of the invention will follow from the detailed description, herein-below.

### BRIEF DESCRIPTION OF THE DRAWINGS

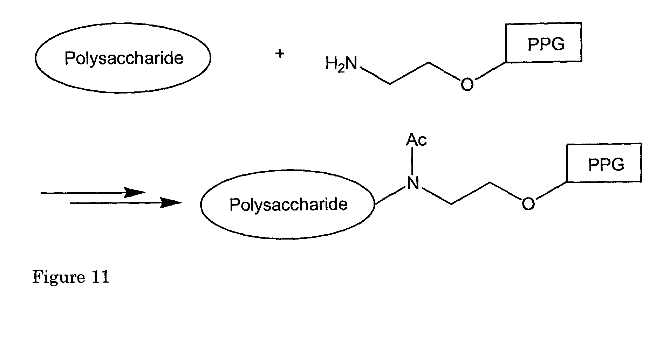

In the drawings:

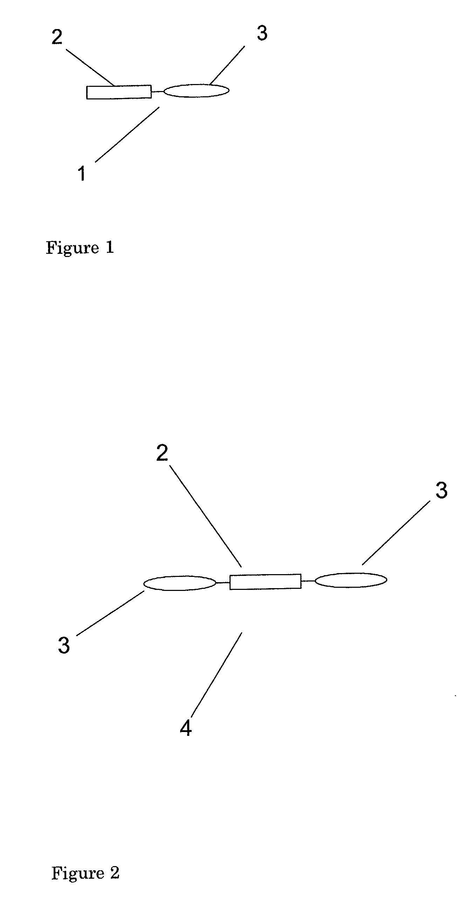

Figure 1 schematically depicts a copolymer molecule of the invention. The A-B block copolymer (1) comprises an A block (3) and a B block (2).
Figure 2 schematically depicts another copolymer molecule of the invention. The A-B-A block copolymer (4) comprises two A blocks (3) and one B block (2).

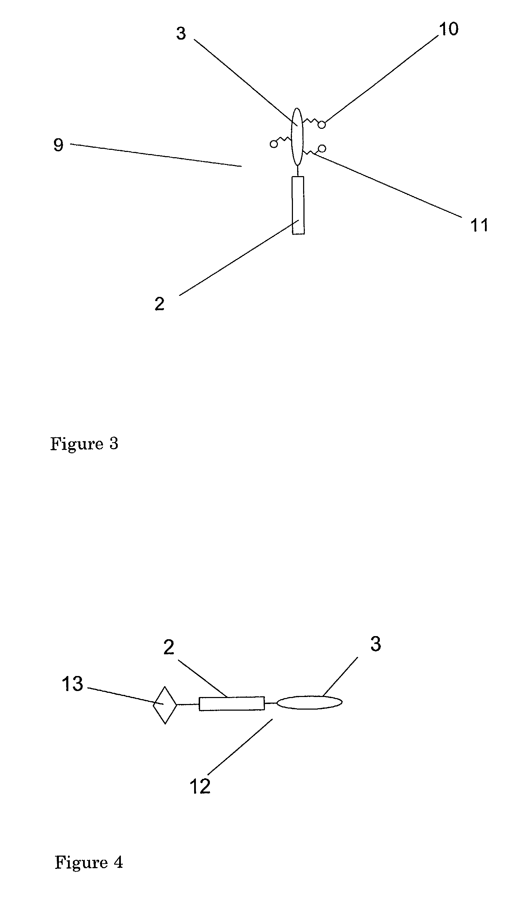

Figure 3 schematically shows a laterally crosslinkable copolymer molecule (9) of the invention. The crosslinkable A-B block copolymer (9) comprises an A block (3) and a B block (2). The A block (3) carries laterally positioned crosslinkable groups (10) attached via spacers (11).
Figure 4 schematically shows a copolymer of the invention which is modified with a ligand. The modified A-B block copolymer (12) comprises an A block (3) and a B block (2). The B block is coupled with a target-recognizing ligand (13).

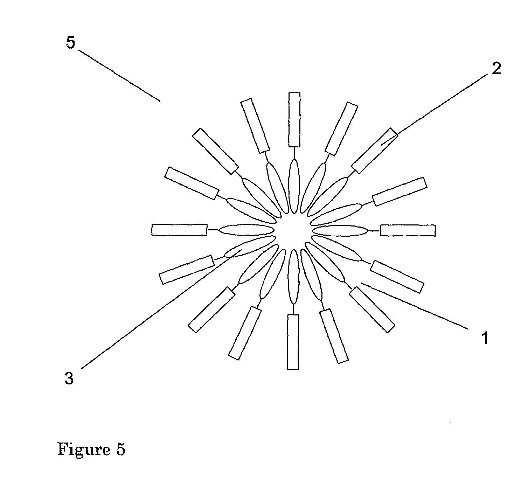

Figure 5 schematically shows a micelle (5) formed by copolymer molecules of the invention. The micelle (5) comprises A-B block copolymers (1) assembled in such a way that the A blocks (3) form the core and the B blocks (2) form the shell of the micelle (5).

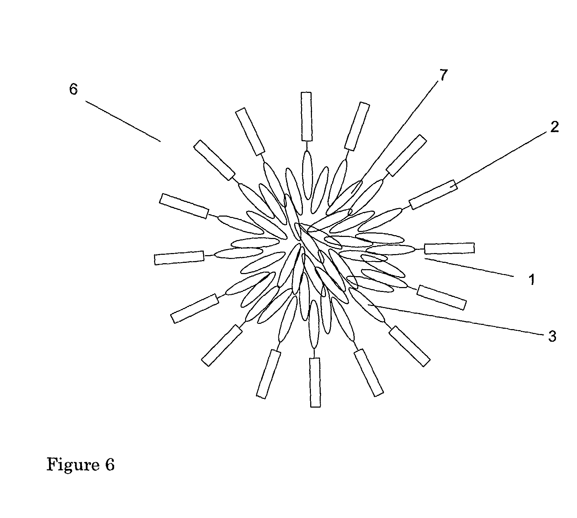

Figure 6 schematically shows an expanded micelle. The expanded micelle (6) is formed by A-B block copolymers (1) assembled in such a way that the A blocks (3) are oriented towards the core, and the B blocks (2) are oriented towards the shell of the expanded micelle (6). The core also contains homopolymers A (7).

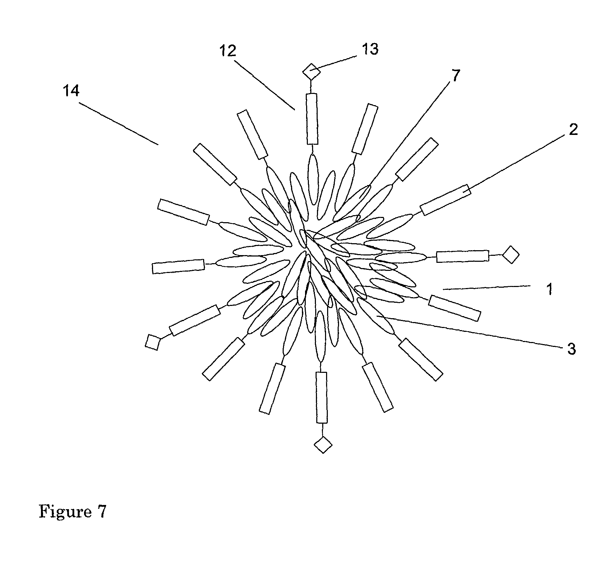

Figure 7 schematically shows a modified expanded micelle. This modified micelle (14) comprises A-B block copolymers (1) and modified A-B block copolymers (12). The modified A-B block copolymer (12) is functionalized with a target-recognizing ligand (13). Both copolymers are assembled in such a way that the A blocks (3) form the core and the B blocks (2) form the shell of the modified expanded micelle (14). The core also contains homopolymers A (7).

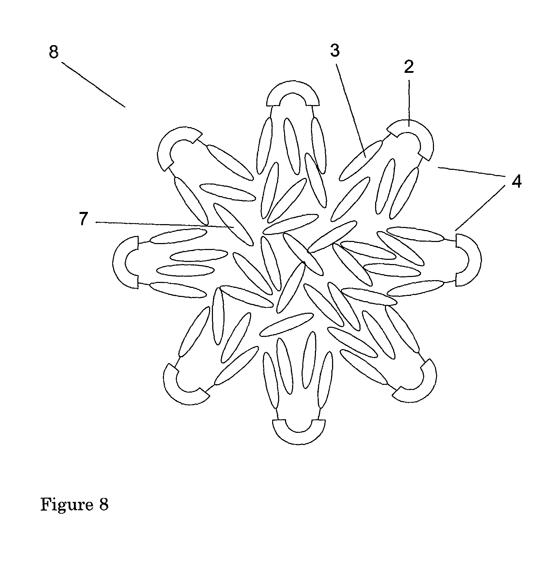

Figure 8 schematically shows another expanded micelle. This expanded micelle (8) comprises A-B-A block copolymers (4) assembled in such a way that the A blocks (3) form the core and the B blocks (2) form the shell of the expanded micelle (8). The core also contains homopolymer A (7).
Figure 9 shows a reaction scheme for the synthesis of a block copolymer by coupling a polysaccharide block and a polyethylene glycol (PEG) block, which coupling is achieved by reacting a glycosylamine derivative of the polysaccharide with a nitrophenyl carbonate of polyethylene glycol at pH 8.5, leading to the formation of a urethane linkage between the polysaccharide and the polyethylene block.
Figure 10 shows a reaction scheme for the synthesis of a block copolymer by coupling a polysaccharide block and a polypropylene glycol (PPG) block, which coupling is achieved by reacting a thioglycolated polysaccharide with a vinyl sulfone derivative of polypropylene glycol at neutral pH, leading to a thioether bridge between the two blocks forming the copolymer.
Finally, Figure 11 shows a reaction scheme for the synthesis of a block copolymer by coupling a polysaccharide block and a polypropylene glycol block, which is achieved by reacting a reducing polysaccharide with an aminoderivative of polypropylene glycol followed by an acetylation of the intermediate Schiff base.

### DETAILED DESCRIPTION OF THE INVENTION

According to a first aspect of the invention, a drug carrier system is provided comprising a plurality of colloidal particles having a core and a shell, said particles being comprised of copolymer molecules, which copolymer comprises at least one A block and at least one B block different from the at least one A block, wherein the at least one A block consists of a polymer unit of a first set of monomers and the at least one B block consists of a second set of monomers, characterized in that the first set of monomers and the second set of monomers are selected in such a way that polymers only consisting of monomers of the first set and polymers only consisting of monomers of the second set are capable of forming an aqueous two-phase system, and in that the A blocks in particles form the core and the B blocks in the particles form the shell.

In the present description and the attached set of claims, the terms "set of monomers" intends to cover the building units of the polymer unit that forms the block. The polymer block does not need to be a homopolymer block, wherein the set of monomers exists only of one type of monomer; the polymer block can also be made of polymerized monomers of two (or more) types. That is, the block can be comprised of a copolymer, a terpolymer or an other type of interpolymer. By way of example, one of the blocks of the block copolymer may be made of ethylene and vinyl alcohol, or of ethylene and vinyl acetate monomers.

In a second aspect, the invention relates to a pharmaceutical composition comprising the colloidal drug carrier system according to any one of the preceding claims.

Further, the invention relates to a block copolymer comprising at least one A block and at least one B block different from the at least one A block, wherein the at least one A block consists of a polymer unit of a first set of monomers and the at least one B block consists of a second set of monomers, characterized in that the first set of monomers and the second set of monomers are selected in such a way that polymers only consisting of monomers of the first set and polymers only consisting of monomers of the second set are capable of forming an aqueous two-phase system, and wherein the at least one A block comprises one or more crosslinkable groups.

In yet a further aspect, the invention relates to the use of the copolymer of the invention as a stabilizer of an aqueous two-phase system.

Also the use of the copolymer of the present invention as a micelle forming agent in an aqueous system forms an aspect of this invention.

Moreover, the present invention relates to an aqueous composition comprising the copolymer of the invention.

In a further embodiment the invention relates to a method for the preparation of a drug carrier system comprising a plurality of colloidal particles, said method comprising the steps of:
(a) preparing an aqueous colloidal solution comprising micelles, said micelles being comprised of block copolymers of the invention; and
(b) crosslinking at least some of the crosslinkable groups; wherein step (b) is carried out after step (a).

In another embodiment, the invention relates to a method for the preparation of a drug carrier system comprising a plurality of colloidal particles, said method comprising the steps of:
(a) preparing an aqueous two-phase system, said system comprising:
   (aa) block copolymers according to the invention;
   (bb) polymers consisting of monomers of the first set;
   (cc) polymers consisting of monomers of the second set; and
   (dd) water;
   wherein the relative amounts of polymers (bb), polymers (cc) and water are selected to induce a phase separation;
(b) crosslinking at least some of the crosslinkable groups;
wherein step (b) is carried out after step (a).

As used herein, a drug carrier system is a pharmaceutical composition or an essential component of a pharmaceutical composition incorporating the drug substance(s) and providing some means of control over the release and/or distribution profile of the drug substance. ]

Colloidal particles refer to particles having a particle size of usually between 1 nanometer and 1000 nanometers in diameter which are suspended in a continuous medium, such as a liquid, a solid, or a gaseous substance. In some cases, particles with a diameter in the lower micrometer range are also included. The preferred mean diameter of the colloidal particles of the invention ranges from about 5 nm to about 50 µm. For some applications, such as for intravenous administration, the particles of the invention have a preferred mean diameter of less than about 1 µm, and more preferably less than 500 nm. The term "particles" is meant to include solid and semi-solid particles of any shape or internal structure which are composed of any material. For example, the term would include structures such as (nano- or micro)capsules, (nano- or micro)spheres, and micelles.

According to the invention, the colloidal particles comprise block copolymers with A blocks oriented towards the core and B blocks oriented towards the shell. As used herein, a block copolymer is a polymer comprising at least a linear sequence of a first set of monomers and at least a linear sequence of a second set of monomers connected to each other. The first set of monomers can consist of only one type of monomers, but also of more than one type of monomers. The second set of monomers can also consist of only one type of monomers or of more than one type of monomers; the second set of monomers differs from the first set of monomers. Preferably, the blocks making up the block copolymer used, comprise the monomers in a linear sequence; block A and block B may however comprise a limited degree of branching.

The copolymers present in the colloidal particles used in the present invention comprise at least A blocks and B blocks. A preferred block sequence is A-B-A, and still more preferred is A-B. However, B-A-B copolymers are also within the scope of the invention, as well as block copolymers wherein the B block is grafted on the A block or *vice versa.*

The copolymer molecules are oriented within the colloidal particles in such a way that the blocks herein defined as A-blocks point toward the core and the B-blocks toward the shell of the particles.

Typically, the coupling of different polymeric blocks is achieved via the introduction of linking groups. Numerous types of such linkages have been described in the literature and can be used in the copolymers of the invention, and they may, e.g., include ester, ether, urethane, amide, thioether, carbonate, and various other types of bonds.

The copolymer is composed of A and B blocks. The A block consists of a polymer unit of a first set of monomers and the at least one B block consists of a second set of monomers. Polymers made of the first set of monomers and polymers of second set of monomers are hydrophilic in nature and typically also water soluble, but are at the same time at least partially incompatible in aqueous solutions. More precisely, the physical mixture of polymers made of the first set and polymers made of the second set of monomers are capable of inducing phase separation in aqueous systems. In other words, the said polymers are capable of forming an aqueous two-phase system, sometimes described as an W/W-emulsion.

Several aqueous two-phase systems have been described in the literature. The most frequently used systems are based on two incompatible nonionic polymers, such as polyethylene glycol/dextran, polypropylene glycol/polyvinyl alcohol, or polyvinylpyrrolidone/methylcellulose. In fact, two-phase systems based on polyethylene glycol/dextran are widely used for the extraction and purification of proteins in the biotechnological industry. Other two-phase systems involve a nonionic polymer and a polyelectrolyte, such as the combinations sodium dextran sulfate/polypropylene glycol and sodium carboxymethyl cellulose/hydroxypropyl dextran. Still other systems use two polyelectrolytes, *e.g.* ovalbumin/casein. Furthermore, some aqueous two-phase systems have been studied which result from the combination of a nonionic polymer and a low molecular weight compound, such as polyethylene glycol/glucose and dextran/propyl alcohol. An extensive list of aqueous two-phase systems was published by Zaslavsky *(cfr.* "Aqueous two-phase partitioning", in: Physical Chemistry and Bioanalytical Applications; Boris Y. Zaslavsky; Marcel Dekker, Inc. New York (1995)), which is incorporated herein by reference to describe suitable two phase systems to define the copolymers used in the present invention.

In accordance with the present invention, the colloidal particles of the drug carrier system of the invention comprise the block copolymer molecules defined above in such a way that the A-blocks are oriented toward the core and the B-blocks toward the shell of the particles. The orientation results from the tendency of the block copolymers to self-assemble, much in the same way as amphiphilic compounds do. Within an aqueous phase, the block copolymer may self-assemble to form micelles, depending on the concentration and chemical nature of the copolymer and on the composition of the aqueous phase. For instance, an A-B block copolymer from a dextran block and a polyethylene glycol block (dex-PEG) may not self-assemble at very low concentrations, but will form micelles at a higher concentration, especially in the presence of polymers comprised only of monomers forming either block A or block B; if block A or block B is made of only one type of monomer, these polymers especially present are homopolymers. By way of example: in an aqueous phase containing polyethylene glycol, dex-PEG molecules are capable of forming micelles in which the dextran blocks form or assemble in the core region and the polyethylene glycol blocks arrange in the peripheral region or corona forming the shell, so that in this case the dextran represents the A block and the PEG the B block, as defined above, in accordance with their orientation within the micelles.

The block copolymers as used in the present invention are also capable of forming self-assembled structures in an aqueous two-phase system. Especially when such copolymers are added to a two-phase system or W/W-emulsion containing polymers which are (essentially) comprised of the sets of monomers of which also blocks A and B are made (the A-polymer and B-polymer, respectively) with A-polymer being enriched in the dispersed inner phase and B-polymer being enriched in the coherent outer phase or the continuous phase, the copolymer will assemble at the interface of the two phase, with its A block extending into the A phase and the B block into the B phase of the emulsion. In this respect, the block copolymers behave like conventional emulsifiers in two-phase systems containing an aqueous and an oil phase. Therefore, the block copolymers defined above may also be used as stabilizers of aqueous two-phase systems, and aqueous systems or compositions containing such block copolymers are within the scope of the invention.

Even though they are stabilized within the aqueous system, such emulsion droplets are not always sufficiently stable for all drug delivery applications. The same is true for the micelles described above. For instance, neither of these self-assembled structures is sufficiently stable to be isolated from the continuous aqueous phase in which it is dispersed. Thus, a preferred embodiment of the invention provides the self-assembled colloidal particles in a chemically stabilized form, *i. e.* with intermolecular crosslinks between the A blocks or the B blocks, or both. Of course, the intermolecular crosslinks are crosslinks in the same particle; it is generally not preferred to have interparticular crosslinks forming agglomerates of particles.

As the A blocks, as defined herein, represent the blocks that are oriented towards the core of the colloid, intermolecular crosslinks between A blocks would lead to core crosslinked micelles, and crosslinks between B blocks result in shell crosslinked micelles. Of course, it is also possible to crosslink both block types to arrive at micelles wherein the core and shell are crosslinked.

To be able to crosslink the micelles or expanded micelles, the respective copolymer blocks A and/or B have to comprise crosslinkable groups or structures. A number of crosslinkable structures are known to prior art, as well as methods to modify polymers in order to introduce such crosslinkable structures or groups. For instance, chemical groups containing carbon-carbon double bonds, such as acrylic or methacrylic groups, have been widely used as crosslinkable substituents of polymers. In the present invention, colloidal particles composed of block copolymers having crosslinked methacrylic or hydroxyethylmethacrylic groups represent a preferred embodiment.

The block copolymers of the invention can carry the crosslinkable groups or structures laterally along the backbone of the block that is to be crosslinked. Alternatively, the chain end of a block can be capped or modified with a reactive group. Laterally crosslinked copolymer micelles differ from chain end fixated assemblies in terms of free volume within the crosslinked region, *i.e.* the core or the shell of the micelle. Furthermore, physically entrapped molecules such as drug substances will typically diffuse more slowly through laterally crosslinked structures. Therefore, the choice which of the copolymer blocks is to be crosslinked, and whether it should be crosslinked laterally or at the chain end, will depend on the specific needs to be served by the colloidal particles, for instance, in terms of the drug load and desired drug release profile.

In case the particles of the present invention are self-assembled block copolymer structures the core thereof being expanded by the presence of an amount A-polymer, core crosslinking can be carried out in such a way that intermolecular crosslinks not only between the A blocks of the copolymer, but also between the A blocks and the A-polymer, and optionally between the different A-polymer chains are formed. This would require that at least some of the A-polymer chains comprise crosslinkable groups or are modified to comprise crosslinkable groups. The crosslinked or polymerized core of a colloidal particle used according to the present invention could thus also be defined as a hydrogel network, or more precisely, a chemically crosslinked hydrogel network.

The usefulness of such hydrogel networks as drug carriers has been described in several documents including WO 98/00170 and WO 98/22093. The advantages of the present invention over these previously disclosed hydrogel particles include the capability of preparing very small particles with nanoscale diameters, and of preparing particles with a shell or surface having tailor-made properties which are different from those of the core, as a result of the use of the block copolymers as described herein.

Particularly preferred colloidal particles with a crosslinked hydrogel core comprise a block copolymer with an A block derived from a polysaccharide and a B block derived from a polyalkylene glycol, further comprising a polysaccharide derivative A-polymer as homopolymer. An example for this embodiment are colloidal particles comprising an A-B block copolymer with a modified dextran as A-block and polyethylene glycol as B-block, further comprising an amount of the modified dextran as homopolymer A to expand the core of the particles. After crosslinking the dextran-derived blocks together with the dextran-derived homopolymer, colloidal particles result which are characterized by a shell of polyethylene glycol, imparting highly desirable surface properties for many drug delivery applications, and a dextran-based hydrogel core, which is highly compatible with many biotechnology-derived drug substances, such as peptides and proteins. Useful modified dextrans include hydroxyethylmethacrylated dextran (dex-HEMA). Another embodiment comprises similar particles with a A-B-A type block copolymer, by virtue of which the particle surfaces are more densely covered with polyethylene glycol.

Optionally, the copolymers used in accordance with the present invention comprise another block type, herein referred to as the C block. Such C blocks may be introduced to impart other desired properties, such as mechanical, physical or chemical properties which may be useful but which do not interfere with the functions of the A and B blocks as described above. For instance, a C block can be introduced as an additional spacer between the A and B block to form an A-C-B triblock copolymer.

For many applications in drug delivery, it is desirable that the colloidal particles and the drug carrier system containing them are biodegradable. For instance, parenteral or pulmonary administration would normally require the particles to be biodegradable, since the body generally cannot excrete such particles that have entered the organism via these routes. As used herein, biodegradability refers to the capability of a substance or chemical group to be chemically or biochemically degraded while being in a physiological environment or by biological means. For instance, enzymatic degradation is a form of biodegradation.

The type and degree of biodegradability that is needed depends on the specific application. For many drug delivery applications, it is desirable that the drug carrier, such as the colloidal particles of the invention, is degradable in biological or systemical fluids without the action of enzymes, such as by hydrolysis. The rate of hydrolysis should be appropriate for the type of administration and the desired release period. Biodegradability should prevent materials that are introduced into the body from accumulating and potentially inducing long-term side effects. For instance, colloidal particles meant to be administered once every week should not remain intact in the body for years, but should degrade within weeks or months to allow the body to eliminate these.

In some preferred embodiments, the colloidal particles of the invention are therefore hydrolysable, showing a rate of hydrolysis which is appropriate for the intended use. In order to be hydrolysable, the particles must comprise a significant amount of hydrolysable material or bonds. Preferably, at least the block copolymer, which is responsible for the integrity of the colloidal particles, should be hydrolysable. More in particular, the hydrolytic degradability should be associated with those blocks of the copolymer which are crosslinked, *i.e.* with the B blocks in the case of shell crosslinked particles, and with the A blocks in the case of core crosslinked particles. For embodiments which represent core crosslinked micelles having an expanded core which also comprise A-polymers, it is preferred that hydrolysability is not only a characteristic of the crosslinked A blocks of the copolymer, but also of the A-polymers present in the core. At least those A-polymer molecules which are crosslinked should be hydrolysable.

The biodegradability of the crosslinked copolymer blocks or of the crosslinked A-polymers can either be a property of the polymeric backbone or of the intermolecular linkages. For instance, if the crosslinked blocks are derived from a polysaccharide such as dextran, the backbones may be enzymatically degradable. A backbone of a peptidic block is even hydrolysable in biological fluids without the action of enzymes. However, in cases wherein the backbone of crosslinked blocks, and optionally of the A-polymer present in the particle core, is not sufficiently degradable in view of the intended use of the particles, it is recommended that the crosslinks are selected to be degradable, or that biodegradable or hydrolysable spacers are present between the backbone and the crosslinking structures.

To serve this purpose, any known methods of introducing biodegradable bonds or spacers into polymers or polymer blocks can be used. As a minimum, the intermolecular crosslink should comprise at least one biodegradable bond. Examples of biodegradable bonds that can easily be introduced between the backbone and the crosslinkable groups of a polymer are ester bonds, lactate bonds, glycolate bonds, carbonate bonds, urethane bonds, anhydride bonds, acetal bonds, hemiacetal bonds, or amide bonds. In a preferred embodiment, biodegradable spacers comprising a hydrolysable ester bond, a hydrolysable amide bond or a hydrolysable carbonate bond are present between the backbone and the crosslinkable group. WO 98/00170, which is incorporated herein by reference for describing suitable biodegradable crosslinking bonds, is an example of prior art disclosing the modification of dextrans with side chains comprising hydrolysable spacers, *i.e.* a carbonate group, or a polylactide and/or -glycolide segment, followed by the introduction of crosslinkable groups, such as acrylic, methacrylic or hydroxyethylmethacrylic groups. The resulting polymers represent useful structures to be incorporated in the block copolymers of the present invention. They also represent useful examples for A-polymers to be incorporated into the core of the colloidal particles of the invention.

After crosslinking, the micelles or expanded micelles are particles which are typically insoluble in aqueous media at physiological conditions. As used herein, insolubility refers to the situation that the particles remain physically intact, even though some of the material contained in the particles may be soluble, and may leach out from the particles. For instance, some non-crosslinked constituents of the particles, *e g.* non-crosslinked block copolymer, A-polymer or excipient, may dissolve in water. Also, the active ingredient that is optionally incorporated in the particles is likely to be water soluble. Nevertheless, due to the polymer network formed by crosslinking the crosslinkable blocks and, optionally, the crosslinkable A-polymer, the integrity of the particles is not affected by leachable components, at least not at physiological conditions. As used herein, physiological conditions refer to the conditions found in physiological fluids of the systems for which the carrier systems of the invention are intended, which generally encompass neutral pH conditions, certain ranges of osmotic pressure, and certain temperature ranges.

The size of the resulting particles is preferably in the range of 5 nm to 50 µm. As used herein, the size or diameter refers to the approximate z-average of a particle as measured by photon correlation spectroscopy. While micelles with a diameter of clearly less than 5 nm are known, the colloidal particles of the present invention are more useful as drug carriers if they are at least 5 nm in diameter. More preferably, the diameter is in the range of 10 nm to 50 µm. The most desirable diameter depends on the intended use of the colloidal particles and the drug carrier system based thereon. If, for instance, the particles are to be used for gene therapy or other types of intracellular delivery of active compounds, particle sizes are preferred which are sufficiently high to allow the efficient incorporation of macromolecular compounds, *i.e.* at least about 20 nm, but which ar, at the same time, sufficiently small to allow the particles to be taken up by the target cell, *i.e.* no more than about 500 nm, more preferably no more than about 300 or even 200 nm, depending on the type of target cell. If the particles are to be used as colloidal carriers for the administration of poorly soluble drug substances with immediate release, the particle size will be selected as a compromise between the optimal diameter for achieving rapid release *(i.e.* as small as possible) and the optimal diameter for allowing a high drug load (typically much larger). As a result, the diameter is preferably selected in the range of about 100 nm and about 500 nm for this type of application. If the intended use involves the long-term release of an incorporated drug substance, such as in an intramuscular or subcutaneous depot formulation, the particle size should be optimized to achieve the desired release profile and a local retention of the carrier system at the site of administration, preferably in the range from about 400 nm and about 50 µm, more preferably in the range from about 500 nm to about 5 µm.

The colloidal particles of the invention are especially useful for the delivery of active ingredients which are difficult to deliver. As used herein, active ingredients are defined as compounds or materials which are either bioactive or which are useful for the detection or characterization of a biological material. Active ingredients include drug substances, diagnostic agents, markers, nutrients, cosmetic agents, preservatives, and pesticides. Preferred active ingredients are drug substances. Drug substances are compounds or compound mixtures that alter the physiological state of an organism. They are most often used for the prevention, diagnosis, and treatment of diseases. The terms "drug" and "drug substance" are used interchangeably herein.

Protein and peptide drugs, but also some polysaccharide drugs, have always presented a major challenge in terms of effective and convenient delivery. These compounds tend to be very instable in gastrointestinal fluids. Furthermore, their physicochemical properties (i. e. their polarity or charge, and molecular size) prevent them from being effectively absorbed through the gastrointestinal mucosa, or through other physiological membranes. Thus, they are typically not sufficiently bioavailable after oral administration, and most therapeutic peptides and proteins need to be injected.

While the use of multiparticulate drug carrier systems has brought about some progress towards a more effective delivery via noninvasive routes of administration, these carriers have most significantly improved the delivery of peptides and proteins by providing controlled release systems which are administered with a reduced frequency. For instance, daily injections of some peptide drugs, such as leuprolide and octreotide, can be replaced by monthly or even less frequent injections of sustained release microparticle formulations.

The drug carrier system of the invention is particularly useful for providing improved controlled release formulations of such drugs. Drug release profiles can be tailored to the needs of the specific application by selecting the appropriate particles size, block copolymer, optionally the A-polymer, spacers, degree of crosslinking, degree or rate of biodegradability etc. Both drug diffusion, *i.e.* through the swollen polymeric network of the crosslinked regions of the particles, and erosion based on the biodegradation of the particles, can be employed as release mechanisms to provide the desired release profile. Another particular advantage of the invention is that the particles can be prepared without the use of organic solvents, to which many of the presently used therapeutic peptides and proteins are sensitive.

The drug carrier system of the invention is also useful for providing improved controlled release formulations of drug substances which are orally bioavailable, but which are sometimes, for the sake of improved compliance or control over the therapy, administered parenterally as controlled release formulations. Preferred drugs in this category are psychoactive drugs including antidepressants and antipsychotic agents, and hormones, such as contraceptive agents.

Another field in which the invention is very useful is the cellular or intracellular delivery of drug substances such as peptides, proteins, or nucleic acid based materials such as genes or oligonucleotides. Especially the delivery of genetic material to target cells, which has traditionally been accomplished with viral vectors, may be improved by the use of colloidal polymeric carriers which are taken up by cells, but which do not have the risks and disadvantages associated with the use of viral vectors. For this use, the colloidal particles of the invention can optionally be further modified. For instance, it seems desirable that the surface of the particles is modified to carry positive charges in order to increase the transfection efficiency.

On the other hand, the invention is also useful for the delivery of conventional drugs, *i.e.* small molecules. In particular, poorly soluble compounds can be delivered by colloidal particles to enhance their dissolution rate, solubility or absorption. Some compounds, such as paclitaxel, are so poorly soluble relative to their dose that it is difficult to formulate them as injectable formulations. Such compounds, when incorporated in the colloidal particles of the invention, can potentially be administered as formulations with a reduced volume to be injected. For the oral administration of such poorly soluble compounds, the colloidal carriers of the invention represent a means of providing rapid drug release and a quick onset of action. Various embodiments can be used to achieve this goal, including simple liquid formulations containing micelles of the block copolymers of the invention comprising a poorly soluble drug substance solubilized therein, as well as crosslinked micelles or crosslinked expanded micelles as described above.

The drug carrier system of the present invention was found especially suitable for substances selected from the group consisting of peptides, proteins, vaccines, nucleic acids, polysaccharides, hormones, poorly water soluble drug substances, psychoactive drug substances and drug substances that are sensitive to organic solvents.

Drug targeting is a drug delivery approach in which a carrier system provides a means of directing its drug load after administration to a specific site of delivery, or site of action. Such targeting or distribution effects can be achieved by virtue of the physical properties of the carrier system (passive drug targeting), such as the delivery of drugs to the macrophages of the RES (reticuloendothelial system) by intravenously injected colloidal carriers, or by specific ligands capable of target recognition and target interaction. For instance, the surface of the colloidal particles in the drug carrier system of the invention can be modified with antibodies that bind specifically to receptors expressed on the target cells. Alternative target-recognizing ligands may be selected, for instance, from the group of peptides, proteins, and carbohydrates.

One way of introducing such surface modifications is to use block copolymers as defined above, which are however further modified at their shell-oriented B blocks to carry a target-recognizing ligand, such as an antibody, peptide, protein, or carbohydrate. Such modified block copolymers may either represent all, or only a fraction of the block copolymer molecules used to prepare the colloidal drug carrier system. The modified copolymers may be anchored chemically in crosslinked micelles or in expanded crosslinked micelles, or they may be anchored physically in the particles, which can be achieved by the use of ligand-modified block copolymers which have no crosslinkable groups.

The colloidal particles are an essential part of the drug carrier system of the invention. The carrier system itself is a pharmaceutical composition which can be administered to an animal, preferably to a human, or it is an essential part of such a pharmaceutical composition. As used herein, a pharmaceutical composition is a physical mixture of a drug substance with at least one excipient or carrier, the composition being formulated and processed to be adapted for administration. Depending on the desired route and mode of administration, different quality requirements must be met by the composition, many of which are set forth in the commonly accepted pharmacopeias, such as the USP, or in guidance documents issued by regulatory agencies such as the FDA. For instance, it is a general requirement for injectable compositions that they must be sterile.

The pharmaceutical compositions comprising the drug carrier system of the invention are adapted for administration via any known route, including oral, peroral, buccal, sublingual, gingival, nasal, transmucosal, ocular, rectal, vaginal, intramuscular, subcutaneous, intracutaneous, intraarterial, intravenous, intratumoral, epidural, intralesional, intraperitoneal, pulmonary, dermal and transdermal administration. Preferred routes of administration within the scope of the invention are intravenous, subcutaneous, intratumoral and intramuscular injection and peroral administration.

The preferred method for preparing the drug carrier system of the invention depends on the specific embodiment with regard to the type of colloidal particles that are needed. The preparation of micelles requires the dispersion of an appropriate block copolymer within the group of polymers described above in an aqueous solution. Preferably, the copolymer is an A-B two-block polymer, and its concentration is selected to be above the critical micelle concentration (CMC). As defined herein, the micelles will be formed with the A blocks being oriented towards the core of the micelles, while the B blocks form the shell or corona. In some cases, the formation of micelles can be facilitated by the presence of a solute that is partially incompatible with the A blocks of the copolymer, such as in the presence of homopolymer B.

The formation of an aqueous micellar solution of the block copolymer is also an important step within the preparation of a drug carrier system based on crosslinked micelles. In this case, a block copolymer is selected which has crosslinkable, or polymerizable groups or structures, such as groups with C-C double bonds. Among the preferred structures are acrylates, diacrylates, oligoacrylates, methacrylates, dimethacrylates, oligomethacrylates, and other biologically acceptable polymerisable groups. For the preparation of core crosslinked micelles, a block copolymer with A blocks having crosslinkable groups are used; for the preparation of shell crosslinked micelles, the B blocks of the copolymer must be crosslinkable. If both the A and B blocks have crosslinkable groups, micelles with crosslinked cores and shells will result.

After the formation of the micellar solution, the micelles are crosslinked. This can be done under any conditions known to prior art which lead to crosslinking. For instance, the crosslinkable groups can be selected to be photopolymerizable, and the crosslinking can be initiated by free radical generation, e. g. through visible or long wavelength ultraviolet radiation (LWUV). Useful photoinitiators are those which can be used to initiate by free radical generation polymerization without cytotoxicity and within a short time frame, minutes at most and most preferably seconds. Useful initiators for LWUV or visible light initiation are ethyl eosin, 2,2-dimethoxy-2-phenyl acetophenone, 2-methoxy-2-phenylacetophenone, other acetophenone derivatives, and camphorquinone. In all these cases, crosslinking and polymerization are initiated by a light-activated free-radical polymerization initiator such as 2,2-dimethoxy-2-phenylacetophenone or a combination of ethyl eosin and triethanol amine, for example. Using such initiators, copolymers may be crosslinked by long wavelength ultraviolet light or by laser light of about 514 nm, for example. Initiation of polymerization is accomplished by irradiation with light at a wavelength of between about 200-700 nm, most preferably in the long wavelength ultraviolet range or visible range, 320 nm or higher, most preferably about 514 nm or 365 nm. Some of the initiators are used with cocatalysts like amines, such as triethanolamine, sulphur compounds, heterocycles such as imidazole, enolates, organometallics, or N-phenyl glycine.

However, other initiation chemistries may be used besides photoinitiation. These include, for example, water and amine initiation schemes with isocyanate or isothiocyanate groups used as the crosslinkable groups. Alternatively, thermal polymerization initiator systems may also be used. Such systems that are unstable at elevated temperatures, such as potassium persulfate, with or without tetramethyl ethylenediamine; benzoylperoxide, with or without triethanolamine; and ammonium persulfate with sodium bisulfite.

The crosslinking of the micelles can be done in the presence or in the absence of the drug substance. In those cases in which it is important to incorporate high drug loads within the core of the crosslinked micelle, it is preferred that the drug is present during the crosslinking step, as long as the drug substance is not adversely affected by the crosslinking reaction. An advantage of this method is that the formation of the particles and the drug loading is accomplished simultaneously. On the other hand, crosslinked micelles can, after their formation in the absence of the drug substance, be incubated with a solution of the drug, which is a useful method for the incorporation of highly sensitive drug substances.

The preparation of micelles with an expanded core is best accomplished by preparing an aqueous two-phase system comprising a block copolymer of the invention. As described above, an aqueous two-phase system can be generated by adding two partially incompatible hydrophilic compounds, such as dextran and polyethylene glycol, to an aqueous phase. An especially useful method for inducing phase separation leading to a two-phase system which comprises an A-B or A-B-A block copolymer of the invention is to combine within an aqueous system an amount of each of the following compounds: (a) the block copolymer, (b) A-polymer, and (c) B-polymer B, wherein the amounts of the A- and B-polymers are selected to induce phase separation. Upon combining the compounds with water, a two-phase system is generated, with one phase being represented by the dispersed droplets which form an aqueous phase enriched with A-polymer, and the other phase being represented by the coherent or outer phase which is enriched with B-polymer. The copolymer assembles at the interface, with the A blocks extending into the dispersed droplets containing the A-polymer, and the B blocks extending into the outer phase containing B-polymer. Provided that a sufficient amount of block copolymer to fully cover the interface area has been selected, the dispersed droplets - having shells of the block copolymer - represent expanded micelles according to the invention.

In some cases, the method can also be practiced in the absence of B-polymer. In other cases, one of the A- and B-polymers may be replaced by another polymer different from A- and B-polymer, provided this other polymer behaves similarly to the one that is replaced. For instance, if A-polymers and polymers D, but not B-polymers and polymers D, are partially incompatible and capable of forming an aqueous two-phase system, expanded micelles may be formed from a two-phase system in which the outer phase is enriched with polymer D, the dispersed droplet phase - *i.e.* the expanded cores of the micelles - is enriched with A-polymer, and the interfaces are occupied by the A-B or A-B-A block copolymer. Similarly, A-polymer can be replaced with a similar polymer, such as polymer E, which is also capable of inducing phase separation in combination with B-polymer.

In order to prepare colloidal particles consisting of crosslinked expanded micelles, an aqueous two-phase system comprising expanded micelles is prepared first, the copolymer being selected to have an A or B block with crosslinkable groups. In a subsequent step, the expanded micelles are crosslinked in a similar fashion as described above for crosslinked micelles without expanded core. Again, either core or shell crosslinking, or both, can be achieved depending on whether the A or the B block of the copolymer, or both blocks, have crosslinkable groups. If also an A-polymer that is present in the expanded core of the micelles has crosslinkable groups, crosslinking will result in a particles core representing a chemical hydrogel, comprising crosslinks between A blocks, between A-polymer molecules, and between A blocks and A-polymer molecules, forming a hydrophilic three-dimensional polymer network.

The block copolymers themselves can be prepared by various routes of synthesis, depending on the chemical nature of the blocks. Some of the presently preferred copolymers, which are composed of at least one polysaccharide block and at least one polyethylene glycol block, can be prepared by coupling a polysaccharide to a polyethylene glycol in one of the following ways.

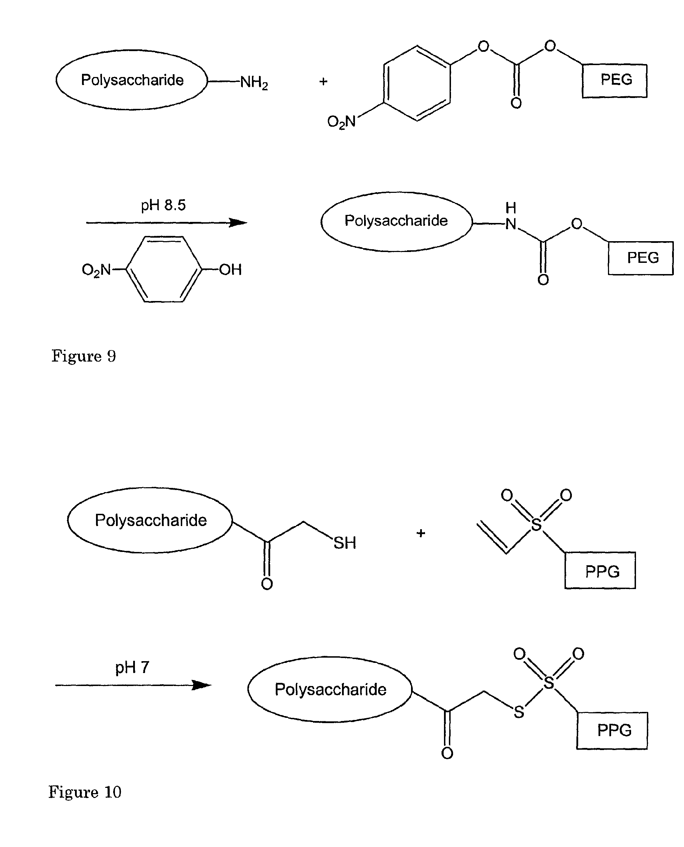

For example, the free anomeric center of a reducing polysaccharide, such as dextran, having a particular reactivity, can be used for the coupling reaction. The advantage of this strategy is that the extensive and time-consuming protection- and deprotection steps for the numerous problematic hydroxyl groups according to the classical carbohydrate chemistry approach can be avoided. Following this line, one of the useful approaches is to first prepare a glycosylamine derivative of a reducing polysaccharide like dextran, such as by treating the polysaccharide with a saturated solution of ammonium hydrogen carbonate. The free primary amino group of the polysaccharide can subsequently be reacted with the nitrophenyl carbonate of polyethylene glycol or polypropylene glycol in borate buffer at pH 8.5, leading to a block copolymer of the polysaccharide and the polyethylen (or polypropylene) glycol in which the two blocks are linked by a urethane linkage (see figure 9).

Alternatively, a thioglycolated polysaccharide, such as thioglycolated dextran, can be coupled with a vinyl sulfone polyethylene glycol or polypropylene glycol to yield a block copolymer with a thioether linkage (see figure 10).

As another alternative, a reducing polysaccharide can be reacted with a polyethylene or polypropylene glycol which has been derivatized to carry a primary amino group, leading to the formation of a Schiff base. The Schiff base should preferably be stabilized by subsequent selective acetylation of the nitrogen of the linkage (see figure 11), which can also be achieved by per-acetylating the copolymer followed by selective or incomplete deacetylation.

In order to synthesize block copolymers with crosslinkable groups, it is advisable to first prepare the block copolymer and then selectively introduce the crosslinkable groups. In fact, it may be possible but rather difficult to couple a polymer such as dextran having crosslinkable groups, such as highly reactive acrylic, methacrylic, or hydroxyethylacrylic groups, with another polymer to form a block copolymer. It is therefore preferred according to the invention to synthesize the block copolymer in the first step, and then introduce the crosslinkable groups.

For example, any of the block copolymers of a polysaccharide and a polyethylene glycol or polypropylene glycol as described above can be reacted with an activated hydroxymethacrylic compound in dimethyl sulfoxide (DMSO) in the presence of dimethyl aminopyridine (DMAP). The ratio of the activated hydroxymethacrylic compound to the polysaccharide used in the reaction will largely control the degree of substitution which is achieved, which will later determine the crosslinking density of the crosslinked micelles of the invention and thereby the density of the polymeric network within the micelles.

## Claims

1. A drug carrier system comprising a plurality of colloidal particles having a core and a shell, said particles being comprised of copolymer molecules, which copolymer comprises at least one A block and at least one B block different from the at least one A block, wherein the at least one A block consists of a polymer unit of a first set of monomers and the at least one B block consists of a second set of monomers, **characterized in that** the first set of monomers and the second set of monomers are selected in such a way that polymers only consisting of monomers of the first set and polymers only consisting of monomers of the second set are capable of forming an aqueous two-phase system, and **in that** the A blocks in particles form the core and the B blocks in the particles form the shell.

2. The drug carrier system of claim 1, having intermolecular crosslinks between at least some of the A blocks in the same particle.

3. The drug carrier system of claim 1 or 2, having intermolecular crosslinks between at least some of the B blocks in the same particle.

4. The drug carrier system of claim 1, 2 or 3, further comprising a polymer consisting of monomers of the first set.

5. The drug carrier system of claim 4, having intermolecular crosslinks between at least some of the A blocks and at least some of the chains of the polymer consisting of monomers of the first set in the same particle.

6. The drug carrier system according to any one of the preceding claims, wherein the A block has a biodegradable backbone.

7. The drug carrier system according to claim 2 or claim 5, having biodegradable spacers between block A and at least some of the intermolecular crosslinks.

8. The drug carrier system of claim 7, wherein the biodegradable spacers comprise a hydrolysable ester bond, a hydrolysable amide bond, or a hydrolysable carbonate bond.

9. The drug carrier system according to any one of the preceding claims, wherein the A block consists of a polymer unit of saccharides or derivatives thereof.

10. The drug carrier system according to claim 9, wherein the saccharide is a dextran, optionally modified with an acrylic, a methacrylic or a hydroxyethylmethacrylic group.

11. The drug carrier system according to any one of the preceding claims, wherein the B block consists of a polymer unit of ethylene glycols.

12. The drug carrier system according to any one of the preceding claims, wherein the colloidal particles are substantially insoluble in an aqueous liquid at physiological conditions.

13. The drug carrier system according to anyone of the preceding claims, wherein the colloidal particles have a mean particle size of between 5 nm and 50 µm.

14. The drug carrier system according to any one of the preceding claims, further comprising an active ingredient and preferably a pharmaceutically active ingredient.

15. A pharmaceutical composition comprising the colloidal drug carrier system according to any one of the preceding claims.

16. A block copolymer comprising at least one A block and at least one B block different from the at least one A block, wherein the at least one A block consists of a polymer unit of a first set of monomers and the at least one B block consists of a second set of monomers, **characterized in that** the first set of monomers and the second set of monomers are selected in such a way that polymers only consisting of monomers of the first set and polymers only consisting of monomers of the second set are capable of forming an aqueous two-phase system, and wherein the at least one A block comprises one or more crosslinkable groups.

17. The copolymer according to claim 16, having the structure A-B or A-B-A.

18. The copolymer of claim 16 or 17, wherein the A block possesses a biodegradable backbone.

19. The copolymer according to any one of claims 16-18, wherein a biodegradable spacer is present between the A block and at least some of the crosslinkable groups.

20. The copolymer of claim 19, wherein the biodegradable spacer comprises a hydrolysable ester bond, a hydrolysable amide bond, or a hydrolysable carbonate bond.

21. The copolymer according to any one of claims 16-20, wherein the A block consists of a block selected from the group consisting of native polysaccharides, modified polysaccharides, polyalkylene oxides, polyalkylene glycols, polyvinyl alcohol, polyvinylpyrrolidone, and proteins.

22. The copolymer of claim 21, wherein A block is comprised of dextran units, optionally modified with acrylic, methacrylic or hydroxyethylmethacrylic groups.

23. The copolymer according to any one of the claims 16-22, wherein the B block is a polyethylene glycol block.

24. The copolymer according to any one of the claims 16-23, further comprising at least one block C which is different from the A block and the B block.

25. The copolymer according to any one of the claims 16-24, wherein the B block further comprises a ligand, such as a target-recognizing peptide, protein, antibody, or carbohydrate.

26. Use of the copolymer according to any one of claims 16-25 as a stabilizer of an aqueous two-phase system.

27. Use of the copolymer according to any one of claims 16-25 as a micelle forming agent in an aqueous system.

28. An aqueous composition comprising the copolymer according to any one of claims 16-25.

29. The composition of claim 28 wherein polymers consisting of monomers of the first set and polymers consisting of monomers of the second set are present in an amount effecting a phase separation between a first aqueous phase rich in polymers consisting of monomers of the first set and a second aqueous phase rich in polymers consisting of monomers of the second set.

30. The composition of claim 29, wherein the second aqueous phase forms the continuous phase of the two-phase system.

31. Method for the preparation of a drug carrier system comprising a plurality of colloidal particles, said method comprising the steps of:
(a) preparing an aqueous colloidal solution comprising micelles, said micelles being comprised of a block copolymer according to any one of claims 16-25; and
(b) crosslinking at least some of the crosslinkable groups; wherein step (b) is carried out after step (a).

32. The method of claim 31, wherein step (b) is carried out in the presence of an active substance.

33. Method for the preparation of a drug carrier system comprising a plurality of colloidal particles, said method comprising the steps of:
(a) preparing an aqueous two-phase system, said system comprising:
(aa) block copolymer according to any one of claims 16-25;
(bb) polymer consisting of monomers of the first set;
(cc) polymer consisting of monomers of the second set; and
(dd) water;
wherein the relative amounts of polymer (bb), polymer (cc) and water are selected to induce a phase separation;
(b) crosslinking at least some of the crosslinkable groups;
wherein step (b) is carried out after step (a).

34. The method of any one of claims 31-33, wherein the aqueous two-phase system comprises a further block copolymer as defined in claim 1.

35. The method of claim 34 wherein at least a part of the B blocks of the block copolymers comprises a target recognizing ligand, such as an antibody, peptide, protein, or carbohydrate.
